# EUROPEAN PATENT APPLICATION

(11) **EP 4 483 781 A1**
(43) Date of publication of application: **01.01.2025**
(21) Application number: 23759810.7
(22) Date of filing: 15.02.2023
(51) Int. Cl.: A61B 3/107, A61B 3/103

(54) **OPTICAL CHARACTERISTIC DISPLAY DEVICE AND OPTICAL CHARACTERISTIC DISPLAY METHOD**

(30) Priority: 22.02.2022 JP 2022026028
(71) Applicant: TOPCON CORPORATION, Tokyo 174-8580 (JP)
(72) Inventor: IKAWA, Fumiya, Tokyo 174-8580 (JP); ISHIHARA, Mutsutaka, Tokyo 174-8580 (JP); HARADA, Akihiro, Tokyo 174-8580 (JP)
(74) Representative: Schmitt-Nilson Schraud Waibel Wohlfrom Patentanwälte Partnerschaft mbB
(86) International application number: PCT/JP2023/005146
(87) International publication number: WO 2023/162809

(57) **Abstract**

An optical characteristic display device and an optical characteristic display method are provided which allow simulating an optical characteristic of a patient's eye corresponding to the patient's eye into which an intraocular lens is inserted. The optical characteristic display device includes an aberration acquiring unit configured to acquire an intraocular aberration, a cornea aberration, and an internal aberration of a patient's eye into which an intraocular lens is inserted, a display controlling unit configured to display an optical characteristic of the patient's eye on a monitor based at least on the intraocular aberration and the internal aberration, a first operating unit configured to receive a changing operation, the changing operation only changing one or more parameters of the internal aberration within the patient's eye, an internal aberration predicting unit configured to predict the internal aberration after the parameter is changed in accordance with the changing operation on the first operating unit, and a re-calculating unit configured to re-calculate the intraocular aberration based on the internal aberration predicted by the internal aberration predicting unit and the cornea aberration acquired by the aberration acquiring unit, and the display controlling unit updates the optical characteristic to be displayed on the monitor based on the internal aberration predicted by the internal aberration predicting unit and the intraocular aberration re-calculated by the re-calculating unit.

## Description

### {Technical Field}

The presently disclosed subject matter relates to an optical characteristic display device and an optical characteristic display method for displaying, on a monitor, an optical characteristic of a patient's eye based on an aberration of the patient's eye.

### {Background Art}

An ophthalmologic device has been known which employs a wavefront sensor to acquire a Hartmann image of a patient's eye by capturing an image of return light from the fundus of the patient's eye (see PTL 1 to PTL 3). This ophthalmologic device analyzes a wavefront aberration of a patient's eye based on a Hartmann image of the patient's eye. For example, this ophthalmologic device calculates, by Zernike analysis on the wavefront aberration, an intraocular aberration of the patient's eye expressed in a Zernike polynomial (Zernike coefficient). Also, the ophthalmologic device acquires an image of an anterior eye part of a patient's eye by capturing an image of return light from the anterior eye part onto which a placido ring is projected, analyzes the anterior eye part image, and calculates a cornea aberration of the cornea of the patient's eye expressed in a Zernike polynomial. Based on the calculation result of the intraocular aberration and the calculation result of the cornea aberration, the ophthalmologic device further calculates an internal aberration of the patient's eye (see PTL 1).

Based on the calculation result of each of the aberrations above of the patient's eye, the ophthalmologic device produces an optical characteristic screen which graphically represents an optical characteristic of the patient's eye, and causes the optical characteristic screen to be displayed on a monitor. This optical characteristic screen includes an aberration map (spherical aberration map, high-order aberration map) of each of the aberrations, a modulation transfer function (MTF), and how the patient's eye can see a Landolt ring (see PTL 2), etc.

Moreover, an ophthalmologic device disclosed in PTL 3 calculates a low-order aberration (aberration that can be corrected by an element such as eyeglasses) and a high-order aberration of a patient's eye based on an intraocular aberration of the patient's eye, which is expressed in a Zernike polynomial. If the high-order aberration is greater than or equal to a threshold, the ophthalmologic device disclosed in PTL 3 calculates a Strehl definition by changing a low-order term of the Zernike polynomial (low-order Zernike coefficient), derives a requirement for a maximum Strehl definition, and determines the low-order aberration amount satisfying the requirement as a correction value for the patient's eye.

### {Citation List}

### {Patent Literature}

{PTL 1} Japanese Patent Application Laid-Open No. 2002-204784
{PTL 2} Japanese Patent Application Laid-Open No. 2002-209854
{PTL 3} Japanese Patent Application Laid-Open No. 2004-089278

### {Summary of Invention}

### {Technical Problem}

When a patient is a cataract patient with astigmatism in the cornea, a toric intraocular lens (IOL) may be inserted into the patient's eye as an intraocular lens to cancel the astigmatism of the cornea in a cataract surgery. In this case, before the cataract surgery, the axis angle of the toric IOL to be inserted into the patient's eye is determined, and guide light is projected onto the patient's eye so as to prevent the axis angle of the toric IOL from being displaced from the astigmatism axis of the patient's eye during the cataract surgery. However, an effect of induced astigmatism caused by incision on the patient's eye during the surgery, an influence of, for example, a displaced axis angle of the toric IOL inserted into the patient's eye after the operation may sometimes require a reoperation.

Preferably, before a reoperation on the patient's eye, how changing a parameter of the internal aberration (aberration of the toric IOL) such as the axis angle, for example, changes the aforementioned optical characteristic of the patient's eye (more specifically, how the patient's eye sees) is simulated, and the simulation result is presented to a person such as the doctor and the patient. However, each of the ophthalmologic devices described in PTL 1 and PTL 2 fails to implement simulation of an optical characteristic of the patient's eye.

On the other hand, PTL 3 discloses that a low-order term of a Zernike polynomial expressing an aberration of a patient's eye is changed. Also, since no element such as a toric IOL is inserted into the patient's eye described in PTL 3, low-order terms of the Zernike polynomial can all be changed with the ophthalmologic device described in PTL 3. However, for the patient's eye into which an intraocular lens such as a toric IOL is inserted, the parameter that can be changed for simulating an optical characteristic of the patient's eye is limited to a parameter that corresponds to the intraocular lens, such as the axis angle, diopter, and position of the toric IOL. Since the ophthalmologic device described in PTL 3 fails to assume the patient's eye into which an intraocular lens such as a toric IOL is inserted, the ophthalmologic device described in PTL 3 fails to implement simulation of an optical characteristic of the patient's eye when a parameter corresponding to the intraocular lens is changed. Therefore, the ophthalmologic device described in PTL 3 fails to implement presenting the simulation result to a person such as the doctor.

The presently disclosed subject matter was made in view of these circumstances, and it is an object of the presently disclosed subject matter to provide an optical characteristic display device and an optical characteristic display method, which allow simulating an optical characteristic of a patient's eye corresponding to the patient's eye into which an intraocular lens is inserted.

### {Solution to Problem}

An optical characteristic display device for achieving the object of the presently disclosed subject matter includes an aberration acquiring unit configured to acquire an intraocular aberration, a cornea aberration, and an internal aberration of a patient's eye into which an intraocular lens is inserted, a display controlling unit configured to display an optical characteristic of the patient's eye on a monitor based at least on the intraocular aberration and the internal aberration among the intraocular aberration, the cornea aberration, and the internal aberration acquired by the aberration acquiring unit, a first operating unit configured to receive a changing operation, the changing operation only changing one or more parameters of the internal aberration within the patient's eye, an internal aberration predicting unit configured to predict the internal aberration after the parameter is changed in accordance with the changing operation on the first operating unit, and a re-calculating unit configured to re-calculate the intraocular aberration based on the internal aberration predicted by the internal aberration predicting unit and the cornea aberration acquired by the aberration acquiring unit, and the display controlling unit updates the optical characteristic to be displayed on the monitor based on the internal aberration predicted by the internal aberration predicting unit and the intraocular aberration re-calculated by the re-calculating unit.

According to this optical characteristic display device, the optical characteristic of a patient's eye when a parameter of an intraocular lens is changed can be predicted (simulated), and the result thereof can be displayed on a monitor.

In the optical characteristic display device according to another aspect of the presently disclosed subject matter, in addition to the optical characteristic, the display controlling unit displays an operation icon on the monitor, the operation icon representing the first operating unit.

In the optical characteristic display device according to another aspect of the presently disclosed subject matter, every time an operation for changing the parameter is repeated in the first operating unit, the prediction of the internal aberration by the internal aberration predicting unit, the re-calculation of the intraocular aberration by the re-calculating unit, and the update processing by the display controlling unit are repeated. Thus, an optimum value of the parameter can be determined.

The optical characteristic display device according to another aspect of the presently disclosed subject matter includes a second operating unit configured to receive an optimization operation for optimizing the parameter, a repetition controlling unit configured to, when the optimization operation is received in the second operating unit, repeat the prediction of the internal aberration by the internal aberration predicting unit and the re-calculation of the intraocular aberration by the re-calculating unit by changing the parameter, an evaluation value calculating unit configured to calculate, for the parameter, an evaluation value being an index of the optimization of the parameter based on the intraocular aberration resulting from the re-calculation for the parameter by the re-calculating unit, and an optimum value determining unit configured to determine an optimum value for the parameter based on a calculation result for the evaluation value for the parameter by the evaluation value calculating unit. Thus, since an optimum value of the parameter can be automatically determined, this optimization can be implemented easily and in a short period of time.

In the optical characteristic display device according to another aspect of the presently disclosed subject matter, the evaluation value is a modulation transfer function or a Strehl definition.

In the optical characteristic display device according to another aspect of the presently disclosed subject matter, the display controlling unit selects the internal aberration and the intraocular aberration corresponding to the optimum value determined by the optimum value determining unit from among the internal aberration and the intraocular aberration for the parameter and updates the optical characteristic to be displayed on the monitor based on the internal aberration and the intraocular aberration corresponding to the optimum value. Thus, a measurement result (simulation result) for the optical characteristic of the patient's eye corresponding to the optimum value can be displayed on a monitor.

In the optical characteristic display device according to another aspect of the presently disclosed subject matter, the display controlling unit displays a value of the parameter on the monitor and changes a display aspect of the value of the parameter to be displayed on the monitor before and after the changing operation in the first operating unit. A tester can recognize the value of the parameter after the changing operation and can further recognize that the optical characteristic of the patient's eye corresponding to the changed parameter is being displayed on the monitor.

In the optical characteristic display device according to another aspect of the presently disclosed subject matter, the aberration acquiring unit includes a first image-capturing system configured to capture a Hartmann image of the patient's eye, an intraocular aberration calculating unit configured to calculate the intraocular aberration based on the Hartmann image captured by the first image-capturing system, a second image-capturing system configured to capture an anterior eye part image of the patient's eye onto which light in a predetermined pattern is projected, a cornea aberration calculating unit configured to calculate the cornea aberration based on the anterior eye part image captured by the second image-capturing system, and an internal aberration calculating unit configured to calculate the internal aberration based on a calculation result for the intraocular aberration by the intraocular aberration calculating unit and a calculation result for the cornea aberration by the cornea aberration calculating unit.

In the optical characteristic display device according to another aspect of the presently disclosed subject matter includes a first storing unit configured to store the intraocular aberration, the cornea aberration, and the internal aberration that are measured in advance, wherein the aberration acquiring unit acquires the intraocular aberration, the cornea aberration, and the internal aberration from the first storing unit. Thus, a time and labor of the tester and a burden on the patient can be reduced.

The optical characteristic display device according to another aspect of the presently disclosed subject matter includes a second storing unit configured to store information that is required for calculating the intraocular aberration, the cornea aberration, and the internal aberration of the patient's eye, including the Hartmann image of the patient's eye and the anterior eye part image of the patient's eye onto which light in the predetermined pattern is projected, and the aberration acquiring unit includes an intraocular aberration calculating unit configured to calculate the intraocular aberration based on the information stored in the second storing unit, a cornea aberration calculating unit configured to calculate the cornea aberration based on the information stored in the second storing unit, and an internal aberration calculating unit configured to calculate the internal aberration based on the information stored in the second storing unit. Thus, the time and labor of the tester and the burden on the patient can be reduced.

In the optical characteristic display device according to another aspect of the presently disclosed subject matter, the parameter includes at least any one of an axis angle, a diopter, and a position of the intraocular lens.

An optical characteristic display method for achieving the object of the presently disclosed subject matter includes an aberration acquiring step of acquiring an intraocular aberration, a cornea aberration, and an internal aberration that are an intraocular aberration, cornea aberration, and an internal aberration of a patient's eye into which an intraocular lens is inserted and that are expressed by a function, a display controlling step of displaying an optical characteristic of the patient's eye on a monitor based at least on the intraocular aberration and the internal aberration among the intraocular aberration, the cornea aberration, and the internal aberration acquired by the aberration acquiring step, an operating step of receiving a changing operation, the changing operation only changing a parameter of the internal aberration within the patient's eye, a predicting step of predicting the internal aberration after the parameter is changed in accordance with the changing operation in the operating step, a re-calculating step of re-calculating the intraocular aberration based on the internal aberration predicted by the predicting step and the cornea aberration acquired by the aberration acquiring step, and an updating step of updating the optical characteristic to be displayed on the monitor based on the internal aberration predicted by the predicting step and the intraocular aberration resulting from the re-calculation processing by the re-calculating step.

### {Advantageous Effect of Invention}

The presently disclosed subject matter allows simulating an optical characteristic of a patient's eye corresponding to the patient's eye into which an intraocular lens is inserted.

### {Brief Description of Drawings}

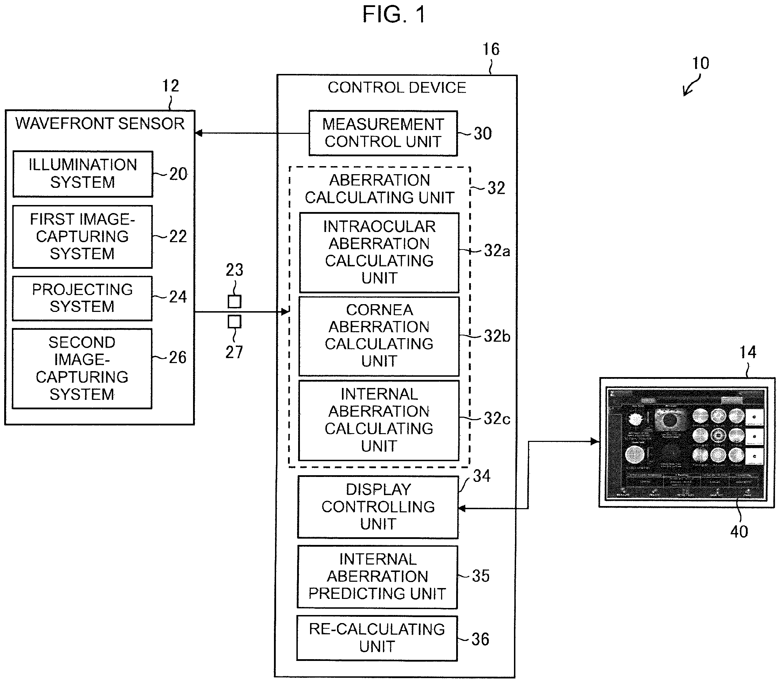

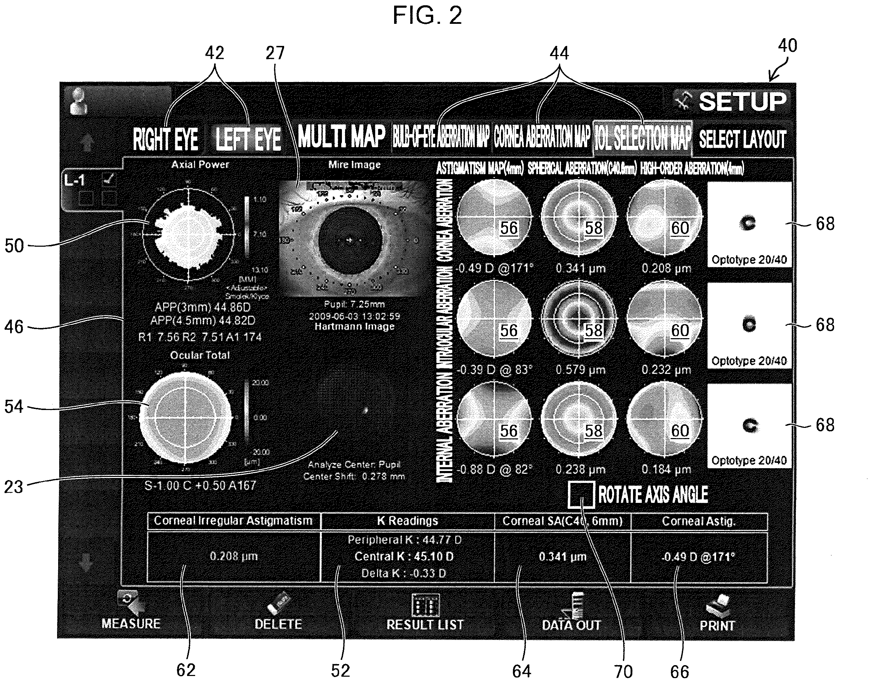

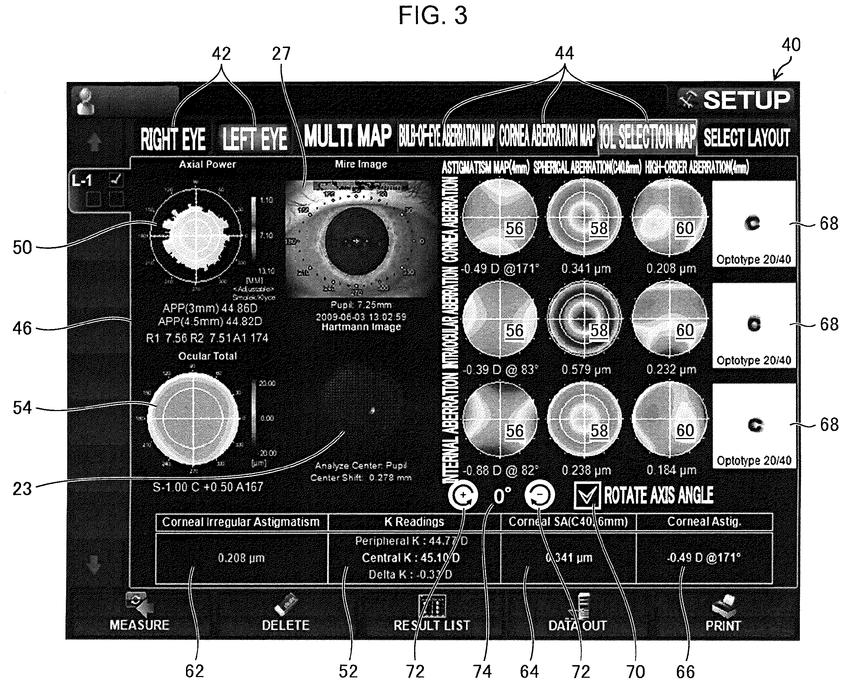

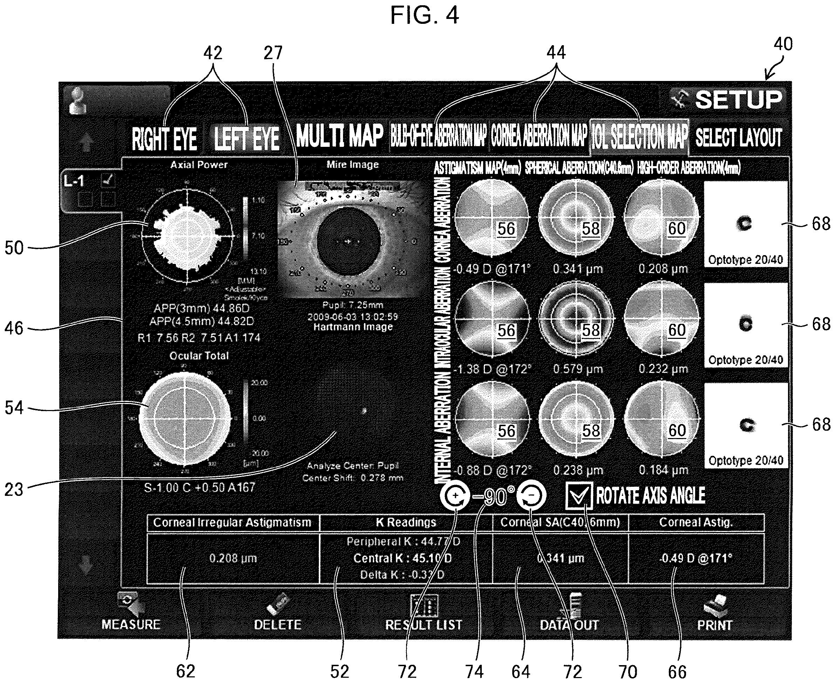

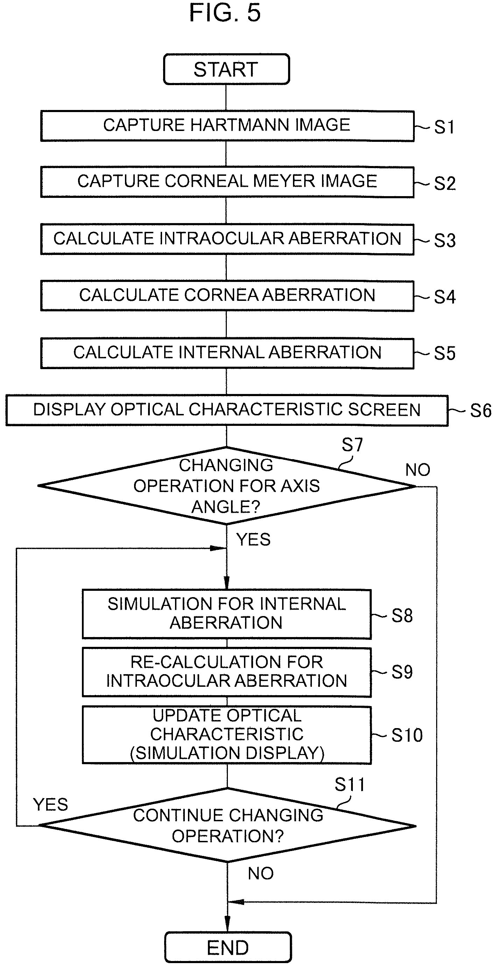

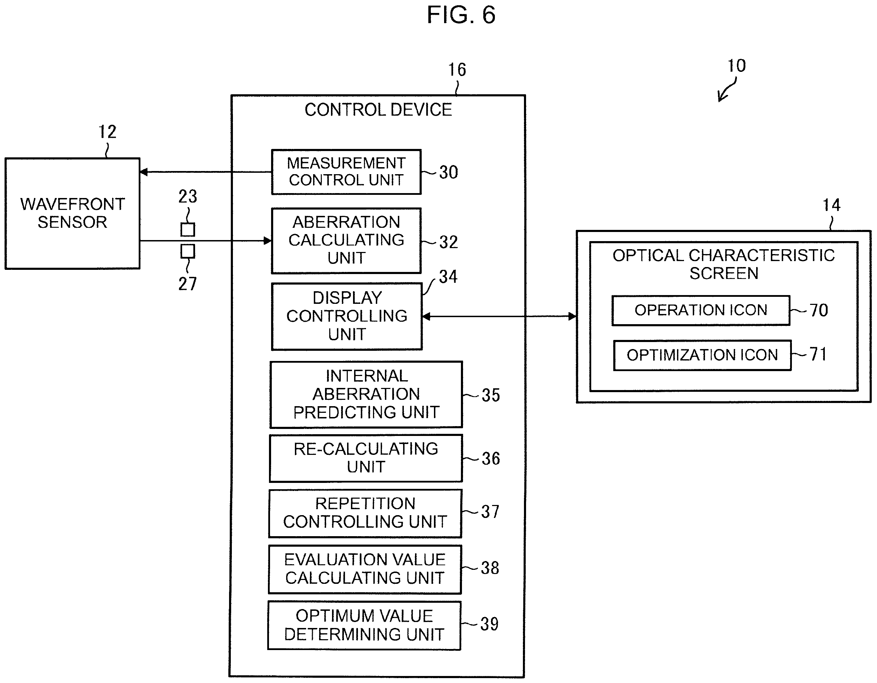

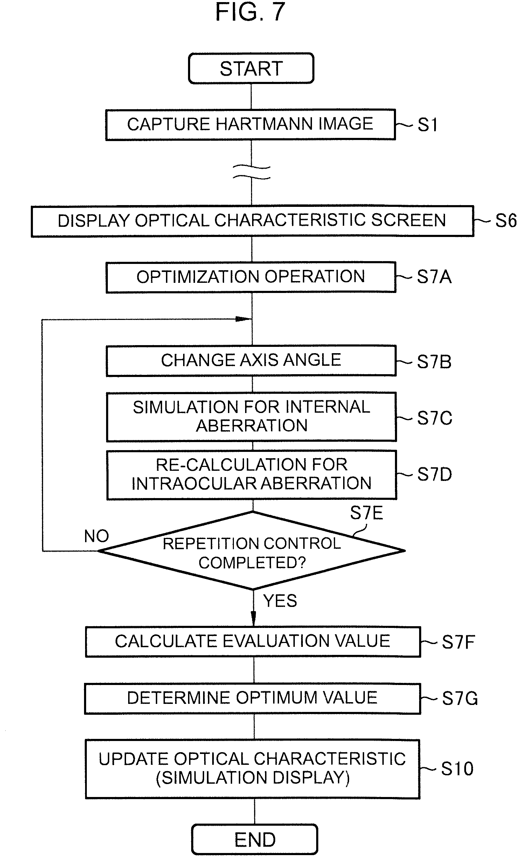

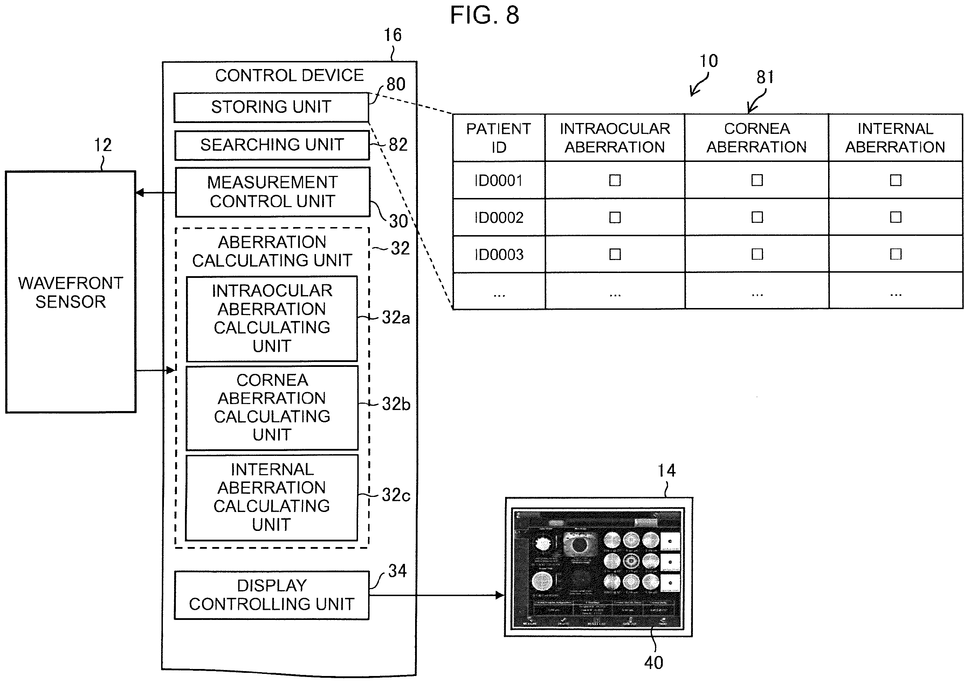

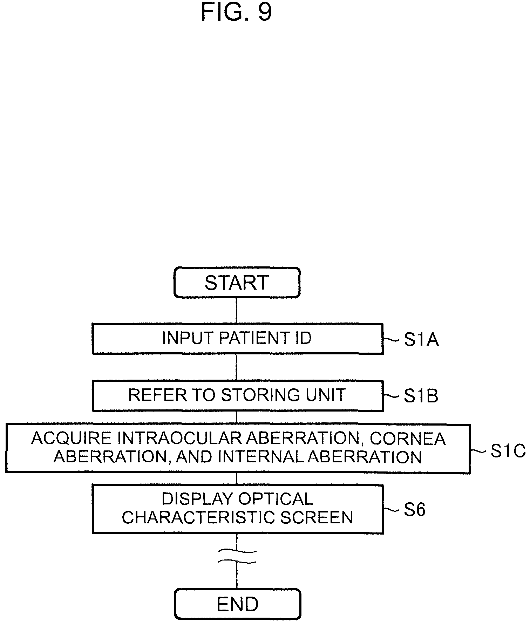

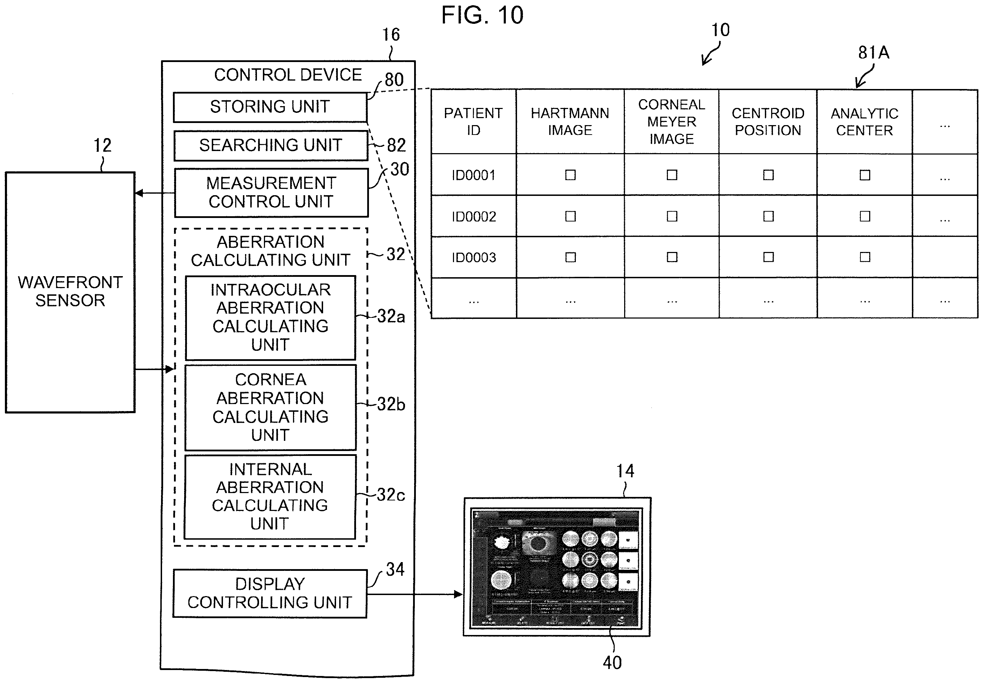

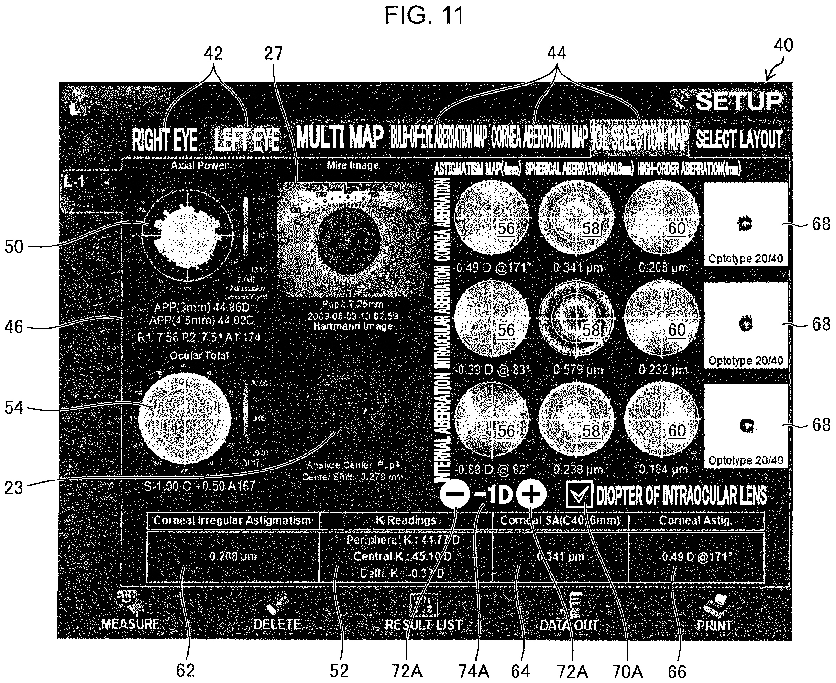

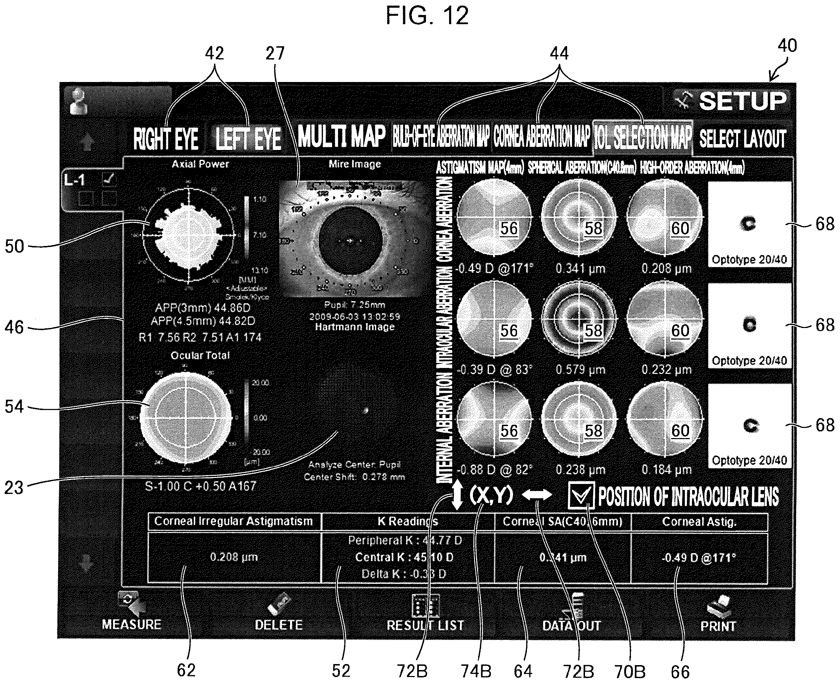

{Figure 1} Figure 1 is a block diagram of an ophthalmologic device according to a first embodiment.
{Figure 2} Figure 2 is a diagram illustrating an example of an optical characteristic screen to be displayed on a monitor.
{Figure 3} Figure 3 is an explanatory diagram illustrating an example of the optical characteristic screen in which a checkbox is checked by a check operation.
{Figure 4} Figure 4 is an explanatory diagram illustrating an example of the optical characteristic screen after a changing operation for changing an axis angle of an internal aberration on an operation icon.
{Figure 5} Figure 5 is a flowchart illustrating a flow of simulation display of an optical characteristic of a patient's eye by the ophthalmologic device according to the first embodiment.
{Figure 6} Figure 6 is a block diagram of an ophthalmologic device according to a second embodiment.
{Figure 7} Figure 7 is a flowchart illustrating a flow of simulation display of an optical characteristic of a patient's eye by the ophthalmologic device according to the second embodiment.
{Figure 8} Figure 8 is a block diagram of an ophthalmologic device according to a third embodiment.
{Figure 9} Figure 9 is a flowchart illustrating a flow of simulation display of an optical characteristic of a patient's eye by the ophthalmologic device according to the third embodiment.
{Figure 10} Figure 10 is a block diagram illustrating an ophthalmologic device according to a fourth embodiment.
{Figure 11} Figure 11 is a diagram illustrating an example of an optical characteristic screen according to a fifth embodiment which allows changing operation of a diopter of an IOL as a parameter of an internal aberration.
{Figure 12} Figure 12 is a diagram illustrating an example of the optical characteristic screen according to the fifth embodiment which allows changing operation of a position of an IOL as the parameter of the internal aberration.

### {Description of Embodiments}

### [First Embodiment]

Figure 1 is a block diagram of an ophthalmologic device 10 according to a first embodiment corresponding to an optical characteristic display device according to the presently disclosed subject matter.

As illustrated in Figure 1, the ophthalmologic device 10 measures an intraocular aberration, a cornea aberration, and an internal aberration of a patient's eye into which, for example, a toric IOL (which is abbreviated as IOL hereinafter) is inserted, produces an optical characteristic screen 40 graphically representing an optical characteristic of the patient's eye based on the measurement results, and causes the optical characteristic screen 40 to be displayed on a monitor 14. Note that a subject is included in a patient, and a subject eye is included in a patient's eye. Also, the intraocular aberration herein refers to an aberration of the whole eye (whole bulb of eye), and the internal aberration refers to an aberration of the IOL excluding the cornea.

Also, the ophthalmologic device 10 has functionality of, before a reoperation for changing an internal aberration parameter of the patient's eye into which an IOL is inserted, predicting an optical characteristic of the patient's eye after the reoperation by a simulation and updating the representation within the optical characteristic screen 40 based on the simulation result. Examples of the internal aberration parameter include an axis angle, a diopter or a position of an IOL. Hereinafter, this embodiment is described with reference to an example in which, before a reoperation for changing the axis angle as an internal aberration parameter, an optical characteristic of the patient's eye is simulated.

The ophthalmologic device 10 has a publicly known corneal topography function and is capable of measuring a corneal shape of a patient's eye. Examples of such a corneal shape include, as illustrated in Figure 2, which is described later, a cornea axial power map 50 in which a curvature of the cornea of a patient's eye is expressed with a diopter or K value information 52 (cornea refractive power).

The ophthalmologic device 10 includes a wavefront sensor 12, the monitor 14, and a control device 16. The wavefront sensor 12 constitutes an aberration acquiring unit of the presently disclosed subject matter along with an aberration calculating unit 32 in the control device 16, which is described later. The wavefront sensor 12 includes an illumination system 20, a first image-capturing system 22, a projecting system 24, and a second image-capturing system 26.

The illumination system 20 and the first image-capturing system 22 are used for measuring (acquiring) an intraocular aberration of the patient's eye. The illumination system 20 radiates illumination light (such as infrared light, for example) to the fundus of the patient's eye. The first image-capturing system 22 captures, through a Hartmann plate, not illustrated, an image of return light from the fundus irradiated with the illumination light from the illumination system 20 and outputs a Hartmann image 23 of the patient's eye to the control device 16. Since the configurations of the illumination system 20 and the first image-capturing system 22 used for capturing the Hartmann image 23 are publicly known technologies (see PTL 1 to PTL 3), specific descriptions thereon are omitted herein.

The projecting system 24 and the second image-capturing system 26 are used for measuring (acquiring) a cornea aberration of a patient's eye. The projecting system 24 projects placido ring light (which may be kerato ring light) corresponding to light in a predetermined pattern of the presently disclosed subject matter onto the cornea of the patient's eye. The second image-capturing system 26 captures an image of return light from the cornea onto which placido ring light is projected by the projecting system 24 and outputs a corneal Meyer image 27 that is an anterior eye part image of the patient's eye to the control device 16. Note that, since the configurations of the projecting system 24 and the second image-capturing system 26 used for capturing the corneal Meyer image 27 are also publicly known technologies (see PTL 1 and PTL 2), specific descriptions thereon are omitted herein. Also, the projecting system 24 and the second image-capturing system 26 may be provided separately from the wavefront sensor 12.

The monitor 14 displays the optical characteristic screen 40 under control of the control device 16. Also, since the monitor 14 is of a so-called touch panel type, it also functions as an operating unit that receives various input operations by a tester. Examples of the input operations include measurement start operations for an intraocular aberration, a cornea aberration, and an internal aberration of a patient's eye, a simulation start operation for an optical characteristic of the patient's eye, and a changing operation for an internal aberration parameter (such as axis angle). An operating unit for those operations may be provided separately from the monitor 14.

The control device 16 includes an element such as an arithmetic circuit including various kinds of processors, and a memory. The various kinds of processors include processors such as a central processing unit (CPU), a graphics processing unit (GPU), an application specific integrated circuit (ASIC), a programmable logic device (such as simple programmable logic devices (SPLD), complex programmable logic device (CPLD), and field programmable gate arrays (FPGA)). Various kinds of functions of the control device 16 may be implemented by one processor or may be implemented by processors of the same kind or of different kinds.

The control device 16 generally controls operations of the wavefront sensor 12 and the monitor 14 based on an input operation input via a touch operation onto the monitor 14. More specifically, the control device 16 controls capturing of the Hartmann image 23 and the corneal Meyer image 27 by the wavefront sensor 12 and displaying of the optical characteristic screen 40 by the monitor 14. The control device 16 executes a control program, not illustrated, to function as a measurement control unit 30, an aberration calculating unit 32, a display control unit 34, an internal aberration predicting unit 35, and a re-calculating unit 36.

The measurement control unit 30 causes the wave front sensor 12 to capture the Hartmann image 23 and the corneal Meyer image 27 based on a measurement start operation input to the monitor 14. Thus, the Hartmann image 23 and the corneal Meyer image 27 are input to the aberration calculating unit 32.

The aberration calculating unit 32 includes an intraocular aberration calculating unit 32a that calculates an intraocular aberration of a patient's eye, a cornea aberration calculating unit 32b that calculates a cornea aberration of the patient's eye, and an internal aberration calculating unit 32c that calculates an internal aberration of the patient's eye.

The intraocular aberration calculating unit 32a analyzes the Hartmann image 23 input from the first image-capturing system 22 and calculates an intraocular aberration of the patient's eye. More specifically, the intraocular aberration calculating unit 32a analyzes the Hartmann image 23, calculates a wavefront aberration of the patient's eye (bulb of eye), and further expresses the wavefront aberration with a mathematical descriptor. For example, the intraocular aberration calculating unit 32a expands, by Zernike analysis, the wavefront aberration of the patient's eye to a Zernike polynomial. Thus, the intraocular aberration calculating unit 32a calculates an intraocular aberration of the patient's eye expressed in a Zernike polynomial (Zernike coefficient).

The cornea aberration calculating unit 32b analyzes the corneal Meyer image 27 input from the second image-capturing system 26 and calculates a cornea aberration expressed in a Zernike polynomial (Zernike coefficient) based on a distortion of a placido ring image that occurs due to an aberration of the cornea of the patient's eye.

The internal aberration calculating unit 32c calculates an internal aberration of a patient's eye which is expressed in a Zernike polynomial (Zernike coefficient), based on a calculation result of an intraocular aberration by the intraocular aberration calculating unit 32a and a calculation result of a cornea aberration by the cornea aberration calculating unit 32b.

Note that, since specific calculation methods for an intraocular aberration, a cornea aberration, and an internal aberration are publicly known technologies (see PTL 1 to PTL 3 (especially PTL 1) and Japanese Patent Application Laid-Open No. 2020-81450), more specific descriptions are omitted herein.

The display control unit 34 controls display on the monitor 14. This display controlling unit 34 produces the optical characteristic screen 40 based on the calculation result of each of the aberrations by the aberration calculating unit 32, the Hartmann image 23 and the corneal Meyer image 27, and the cornea axial power map 50 and the K value information 52 which are acquired in advance and causes the optical characteristic screen 40 to be displayed on the monitor 14.

Figure 2 is a diagram illustrating an example of the optical characteristic screen 40 to be displayed on the monitor 14. This optical characteristic screen 40 includes a left/right eye switching button 42, a layout switching button 44, and a display area 46.

The left/right eye switching button 42 is used for a switching operation between the left eye and the right eye of a patient for displaying an optical characteristic thereof. The display controlling unit 34 switches display for the optical characteristic between the left eye and the right eye to be displayed on the optical characteristic screen 40 in accordance with a left/right eye switching operation on the left/right eye switching button 42 by the tester.

The layout switching button 44 is used for a switching operation for the layout of an optical characteristic of the patient's eye to be displayed on the optical characteristic screen 40. Examples of the type of layout include a "bulb-of-eye aberration map" displaying a bulb-of-eye aberration map, a "cornea aberration map" displaying a cornea aberration map or an "IOL selection map" to be used for a simulation display of an optical characteristic of the patient's eye before a reoperation. In accordance with a selection operation on the layout switching button 44, the display controlling unit 34 causes an optical characteristic of the patient's eye corresponding to the selection operation to be displayed in the display area 46.

Note that the following description according to this embodiment assumes that the "IOL selection map" is selected. The display controlling unit 34 produces graphical representations indicating optical characteristics based on the calculation results of the intraocular aberration (wavefront aberration), cornea aberration, and internal aberration of the patient's eye by the aberration calculating unit 32 and causes them to be displayed in the display area 46 on the "IOL selection map". Also, the display controlling unit 34 causes the Hartmann image 23 and the corneal Meyer image 27 used for the calculation (analysis) by the aberration calculating unit 32 and the cornea axial power map 50 and the K value information 52 acquired in advance by a corneal topography function of the ophthalmologic device 10 to be displayed in the display area 46 on the "IOL selection map".

In the display area 46 on the "IOL selection map", there are displayed, as graphical representations of the optical characteristics of the patient's eye, a "bulb-of-eye total aberration map 54", an "astigmatism map 56", a "spherical aberration map 58", a "high-order aberration map 60", "cornea high-order aberration information 62", "cornea spherical aberration information 64", "cornea astigmatism information 66", and a "Landolt ring simulation image 68." Also, a "checkbox 70" is displayed in the display area 46.

The bulb-of-eye total aberration map 54 displays a total refractive state of a patient's eye. The astigmatism map 56 is a map of astigmatism of the patient's eye and illustrates a cornea astigmatism, a bulb-of-eye astigmatism, and an inner astigmatism in order from the top. The spherical aberration map 58 is a map of a spherical aberration of the patient's eye and illustrates a cornea spherical aberration, a bulb-of-eye spherical aberration, and an inner spherical aberration in order from the top. The high-order aberration map 60 is a map of high-order aberration of the patient's eye and illustrates a cornea high-order aberration, a bulb-of-eye high-order aberration, and an inner high-order aberration in order from the top. The display controlling unit 34 produces the bulb-of-eye total aberration map 54, the astigmatism map 56, the spherical aberration map 58, and the high-order aberration map 60 based on calculation results of an intraocular aberration, a cornea aberration, and an internal aberration of a patient's eye by a publicly known method (see PTL 1, for example) and causes them to be displayed in the display area 46.

The cornea high-order aberration information 62 displays a value of a high-order aberration of the cornea of the patient's eye. The cornea spherical aberration information 64 displays a value of a spherical aberration of the cornea of the patient's eye. The cornea astigmatism information 66 displays a value of a diopter of astigmatism of the cornea of the patient's eye. The display controlling unit 34 calculates the cornea high-order aberration, the cornea spherical aberration, and the diopter of cornea astigmatism by a publicly known method based on the calculation result of the cornea aberration of the patient's eye and causes the calculation results, the cornea high-order aberration information 62, the cornea spherical aberration information 64, and the cornea astigmatism information 66, to be displayed in the display area 46.

The Landolt ring simulation image 68 is an image representing how a Landolt ring is seen by a patient's eye. The display controlling unit 34 produces the Landolt ring simulation image 68 by a publicly known method (see PTL 1 and PTL 2, for example) based on the wavefront aberration of the patient's eye, which is calculated from the Hartmann image 23 by the intraocular aberration calculating unit 32a and causes it to be displayed in the display area 46.

The checkbox 70 receives a start operation, more specifically, a check operation, for starting a simulation display of an optical characteristic of the patient's eye when the axis angle of an internal aberration corresponding to the axis angle of the IOL inside the patient's eye is changed.

Figure 3 is an explanatory diagram illustrating an example of the optical characteristic screen 40 when the checkbox 70 is checked by a check operation. As illustrated in Figure 3, when the checkbox 70 is checked by the check operation of a tester, the display controlling unit 34 causes an operation icon 72 and axis angle information 74 to be displayed within, for example, the display area 46 (which may be outside the display area 46 alternatively) on the optical characteristic screen 40.

The operation icon 72 corresponds to the first operating unit of the presently disclosed subject matter and receives a changing operation for changing a parameter of an internal aberration corresponding to an aberration of the IOL, that is, a changing operation for the axis angle of the internal aberration here. This changing operation for the axis angle includes a rotation operation for rotating the axis angle in a clockwise direction and a rotation operation for rotating it in a counter-clockwise direction.

The operation icon 72 is configured to implement the changing operation for the axis angle of the internal aberration. The operation icon 72 is not particularly limited in terms of a number, shape and display aspect thereof. For example, a pop-up display of the operation icon 72 may be provided on the optical characteristic screen 40 (in the display area 46). Also, how the axis angle is input to the operation icon 72 is not particularly limited and may include a touch operation, a motion operation, a drag operation or a direct input with a keyboard.

The axis angle information 74 displays a value of the axis angle of the internal aberration (corresponding to a value of the parameter of the presently disclosed subject matter). Here, the axis angle of the current (before a reoperation) internal aberration (of the IOL inside the patient's eye) is assumed to 0 degrees. When the axis angle is rotated by α degree(s) in the clockwise direction from 0 degrees by changing the axis angle on the operation icon 72 by the changing operation, a rotated amount is referred to as +α degree(s). Conversely, when the axis angle is rotated by α degree(s) in the counter-clockwise direction, the rotated amount is referred to as -α degrees.

Referring back to Figure 1, when the axis angle of the internal aberration is changed by a changing operation on the operation icon 72, the internal aberration predicting unit 35 predicts an internal aberration after the changing operation by a simulation. More specifically, the internal aberration predicting unit 35 refers to the Zernike polynomial expressing an internal aberration calculated by the internal aberration calculating unit 32c. Then, the internal aberration predicting unit 35 changes the second-order term corresponding to the axis angle of the internal aberration within the Zernike polynomial that expresses the internal aberration. Thus, the internal aberration predicting unit 35 can simulate (predict) the internal aberration after the axis angle of the internal aberration is changed. Subsequently, the internal aberration predicting unit 35 repeats the simulation of the internal aberration every time the axis angle of the internal aberration is changed by the changing operation on the operation icon 72.

When the internal aberration predicting unit 35 predicts the internal aberration through the simulation with respect to the internal aberration, the re-calculating unit 36 re-calculates, by re-calculation processing, an intraocular aberration of the patient's eye based on the internal aberration of the patient's eye simulated by the internal aberration predicting unit 35 and the cornea aberration calculated by the cornea aberration calculating unit 32b. Thus, the intraocular aberration after the change of the axis angle of the internal aberration is simulated. Subsequently, the re-calculating unit 36 re-calculates, by re-calculation processing, an intraocular aberration, that is, simulation with respect to an intraocular aberration every time the internal aberration predicting unit 35 predicts a new internal aberration through the simulation.

Figure 4 is an explanatory diagram illustrating an example of the optical characteristic screen 40 after an axis angle of an internal aberration is changed by the changing operation on the operation icon 72. As illustrated in Figure 4, after the simulation with respect to the internal aberration by the internal aberration predicting unit 35 and the re-calculation processing for the intraocular aberration by the re-calculating unit 36, the display controlling unit 34 updates, based on the results thereof, the display of the optical characteristic of the patient's eye corresponding to the internal aberration and intraocular aberration within the display area 46 and displays the simulation result of the internal aberration and the intraocular aberration. Here, the optical characteristic of the patient's eye corresponding to the internal aberration and the intraocular aberration refers to a map, an image and a measurement value produced or calculated based at least on one of the internal aberration and the intraocular aberration. For example, according to this embodiment, the bulb-of-eye total aberration map 54, the astigmatism map 56, the spherical aberration map 58, and the high-order aberration map 60 excluding the cornea aberration and the Landolt ring simulation image 68 are updated (simulation display).

Also, the display controlling unit 34 changes the display aspect of the axis angle information 74 before and after a changing operation for the axis angle of the internal aberration. For example, the change is not particularly limited if the difference from the axis angle information 74 before a changing operation for the axis angle is clear, such as changing the display color, changing the font, or changing the size of the axis angle information 74 before and after the changing operation.

### [Effects of First Embodiment]

Figure 5 is a flowchart illustrating a flow of simulation display of an optical characteristic of a patient's eye by the ophthalmologic device 10 of the first embodiment having the aforementioned configuration, according to the optical characteristic display method of the presently disclosed subject matter.

As illustrated in Figure 5, in response to a measurement start operation on the monitor 14 by a tester with the patient's face having undergone an IOL insert surgery supported by a face support, not illustrated, the measurement control unit 30 operates after a publicly known alignment. Then, the measurement control unit 30 controls the corresponding units of the wavefront sensor 12 so as to cause capturing of the Hartmann image 23 (step S1) and capturing of the corneal Meyer image 27 (step S2) of the patient's eye. Thus, the Hartmann image 23 and the corneal Meyer image 27 are input from the wavefront sensor 12 to the aberration calculating unit 32.

Then, the intraocular aberration calculating unit 32a analyzes the Hartmann image 23 and calculates an intraocular aberration of the patient's eye, which is expressed in a Zernike polynomial (step S3), and the cornea aberration calculating unit 32b analyzes the corneal Meyer image 27 and calculates a cornea aberration of the patient's eye, which is expressed in a Zernike polynomial (step S4). Then, the internal aberration calculating unit 32c calculates an internal aberration of a patient's eye which is expressed in a Zernike polynomial, based on a calculation result of an intraocular aberration by the intraocular aberration calculating unit 32a and a calculation result of a cornea aberration by the cornea aberration calculating unit 32b (step S5). Here, step S 1 through step S5 correspond to the aberration acquiring step of the presently disclosed subject matter.

When the calculation of each kind of aberration of the patient's eye completes, the display controlling unit 34 produces the optical characteristic screen 40 as illustrated in Figure 2 as described above based on the calculation result of each of the aberrations, the Hartmann image 23 and the corneal Meyer image 27, and the cornea axial power map 50 and the K value information 52 which are acquired in advance and causes the optical characteristic screen 40 to be displayed on the monitor 14 (step S6, corresponding to the display control step of the presently disclosed subject matter).

For simulation display for an optical characteristic of the patient's eye when the axis angle is changed as a parameter of the internal aberration within the patient's eye, the checkbox 70 within the display area 46 on the optical characteristic screen 40 is checked by the check operation of a tester, as illustrated in Figure 3 described above. Thus, the display controlling unit 34 causes the operation icon 72 and the axis angle information 74 to be displayed within the display area 46.

Then, when the tester starts the changing operation for the axis angle of the internal aberration on the operation icon 72 (step S7, corresponding to the operating step of the presently disclosed subject matter), the internal aberration predicting unit 35 operates. The internal aberration predicting unit 35 changes the second-order term corresponding to the axis angle of the internal aberration in the Zernike polynomial that expresses the internal aberration calculated by the internal aberration calculating unit 32c to predict, by a simulation operation, an internal aberration of the patient's eye when the axis angle is changed (step S8, corresponding to the predicting step of the presently disclosed subject matter).

Then, based on the simulation result (prediction result) for the internal aberration by the internal aberration predicting unit 35 and the cornea aberration calculated by the cornea aberration calculating unit 32b, the re-calculating unit 36 re-calculates, by a re-calculation processing, an intraocular aberration of the patient's eye to simulate an intraocular aberration of the patient's eye when the axis angle of the internal aberration is changed (step S9, corresponding to the re-calculating step of the presently disclosed subject matter).

When the re-calculation processing for an intraocular aberration by the re-calculating unit 36 completes, the display controlling unit 34 updates, by simulation display, the display of the corresponding optical characteristic within the display area 46 as illustrated in Figure 4 described above based on the simulation result for an internal aberration by the internal aberration predicting unit 35 and the re-calculation result for an intraocular aberration by the re-calculating unit 36 (step S10). Here, step S10 corresponds to the updating step of the presently disclosed subject matter. Thus, the tester (doctor) and the patient can check how the way of seeing by the patient's eye changes when where the axis angle of the internal aberration is changed.

At the same time, the display controlling unit 34 changes the display aspect of the axis angle information 74 in accordance with the changing operation for the axis angle of the internal aberration on the operation icon 72. Thus, the tester can recognize the axis angle of the internal aberration after the changing operation and can further recognize that the simulation display is being provided for the optical characteristic of the patient's eye corresponding to the changed axis angle.

Subsequently, the processing from step S8 to step S10 as described above is repeated every time the axis angle of an internal aberration is changed by the changing operation on the operation icon 72 (step S11). Thus, the doctor can determine an optimum value of the axis angle of the internal aberration with reference to the simulation display for the optical characteristic of the patient's eye that is updated in the display area 46 every time a changing operation is performed.

As described above, the ophthalmologic device 10 of the first embodiment allows simulating an internal aberration and an intraocular aberration of a patient's eye when the axis angle of the internal aberration is changed and simulation display of an optical characteristic of the patient's eye based on the result of the simulation. This allows simulation of an optical characteristic of the patient's eye corresponding to a parameter change for the internal aberration such as the axis angle of the internal aberration. As a result, there can be provided determination information usable for determining by the doctor and the patient whether a reoperation is required or not. Also, there can be provided determination information usable for determining how much the doctor should rotate the axis angle of the internal aberration (of the IOL) for the reoperation.

### [Second Embodiment]

Figure 6 is a block diagram of an ophthalmologic device 10 according to a second embodiment of the presently disclosed subject matter. According to the first embodiment, the doctor determines the optimum value for the axis angle of the internal aberration by changing, by a changing operation, the axis angle of the internal aberration. On the other hand, the ophthalmologic device 10 of the second embodiment has functionality of optimization of the axis angle of the internal aberration, and this optimum value can be automatically determined.

As illustrated in Figure 6, the ophthalmologic device 10 of the second embodiment has a configuration that is basically the same as that of the ophthalmologic device 10 of the first embodiment except that the display controlling unit 34 causes an optimization icon 71 to be displayed within the optical characteristic screen 40 and the control device 16 further functions as a repetition controlling unit 37, an evaluation value calculating unit 38, and an optimum value determining unit 39. Thus, like references are given to those having the same function or configuration as that of each of the aforementioned embodiments, and repetitive description is omitted.

The optimization icon 71 corresponds to the second operating unit of the presently disclosed subject matter and receives an optimization operation for optimizing the axis angle of the internal aberration. In response thereto, the repetition controlling unit 37, the evaluation value calculating unit 38, and the optimum value determining unit 39 of the control device 16 operate.

The repetition controlling unit 37 changes a setting for the axis angle of an internal aberration and, at the same time, repeatedly executes a simulation for an internal aberration by the internal aberration predicting unit 35 and re-calculation processing for an intraocular aberration by the re-calculating unit 36. Thus, an internal aberration and an intraocular aberration are simulated for each axis angle of the internal aberration.

Based on the intraocular aberration as a result of the re-calculation processing for each axis angle of the internal aberration by the re-calculating unit 36 in the aforementioned repetition control, the evaluation value calculating unit 38 calculates an evaluation value that acts as an indicator for optimization of the axis angle of the internal aberration for each axis angle of the internal aberration. The evaluation value may include a modulation transfer function (MTF) that acts as an indicator of resolving power or a Strehl definition that is known as an evaluation method for a point image. Since a method for calculating an MTF or a Strehl definition from an intraocular aberration (wavefront aberration) of the patient's eye is publicly known technology (see PTL 1 to PTL 3), more specific descriptions are omitted herein.

Based on the evaluation value calculation result for each axis angle of the internal aberration by the evaluation value calculating unit 38, the optimum value determining unit 39 determines the axis angle of the internal aberration that provides a maximum evaluation value as an optimum value and outputs the determination result for the optimum value to the display controlling unit 34.

Based on the optimum value for the axis angle of the internal aberration determined by the optimum value determining unit 39, the display controlling unit 34 of the second embodiment selects an internal aberration and an intraocular aberration corresponding to the optimum value from the internal aberrations and intraocular aberrations for the axis angles of the internal aberration acquired in the aforementioned repetition control. Then, based on the internal aberration and the intraocular aberration corresponding to the optimum value, the display controlling unit 34 updates the display of the corresponding optical characteristic within the display area 46 by a simulation display.

When the optimum value determining unit 39 determines an optimum value for the axis angle of the internal aberration, the internal aberration predicting unit 35 may simulate the internal aberration corresponding to the optimum value and the re-calculating unit 36 may re-calculate an intraocular aberration.

Figure 7 is a flowchart illustrating a flow of simulation display of an optical characteristic of a patient's eye by the ophthalmologic device 10 according to the second embodiment. Since the processing from step S1 to step S6 is the same as that of the aforementioned first embodiment described with reference to Figure 5 that is described above, specific description is omitted here.

For optimization of the axis angle of the internal aberration, an optimization operation is inputted via a touch operation on the optimization icon 71 by a tester within the optical characteristic screen 40 (step S7A). When the optimization operation is inputted, the repetition controlling unit 37, evaluation value calculating unit 38, and optimum value determining unit 39 in the control device 16 operate.

Then, the repetition controlling unit 37 starts repetition control so as to cause changing of the axis angle of the internal aberration (step S7B), simulation for an internal aberration by the internal aberration predicting unit 35 (step S7C), and re-calculation processing for an intraocular aberration by the re-calculating unit 36 (step S7D) to be executed repeatedly (step S7E). Thus, the internal aberration and intraocular aberration are simulated for each axis angle of the internal aberration.

Based on the intraocular aberration as a result of the re-calculation processing for each axis angle of the internal aberration by the re-calculating unit 36, the evaluation value calculating unit 38 calculates an evaluation value for each axis angle of the internal aberration. An evaluation value by the evaluation value calculating unit 38 may be calculated after completion of the aforementioned repetition control as illustrated in Figure 7 or may be calculated every time the re-calculating unit 36 re-calculates an intraocular aberration during the execution of the repetition control.

Upon completion of the calculation of all evaluation values for axis angles of the internal aberration by the evaluation value calculating unit 38, the optimum value determining unit 39 determines an optimum value for the axis angle of the internal aberration based on the evaluation value calculation results and outputs the determination result for the optimum value to the display controlling unit 34 (step S7G).

Then, based on the internal aberration and the intraocular aberration corresponding to the optimum value of the axis angle of the internal aberration determined by the optimum value determining unit 39, the display controlling unit 34 updates the display of the corresponding optical characteristic within the display area 46 by a simulation display like the first embodiment (step S10).

As described above, since the ophthalmologic device 10 of the second embodiment is capable of automatically optimizing the axis angle of the internal aberration, this optimization can be implemented easily and in a short period of time.

### [Third Embodiment]

Figure 8 is a block diagram of an ophthalmologic device 10 according to a third embodiment. Figure 9 is a flowchart illustrating a flow of simulation display of an optical characteristic of a patient's eye by the ophthalmologic device 10 according to the third embodiment.

According to the ophthalmologic device 10 of the aforementioned embodiments, the Hartmann image 23 and the corneal Meyer image 27 of a patient's eye are captured by the wavefront sensor 12, and these Hartmann image 23 and corneal Meyer image 27 are analyzed by the aberration calculating unit 32 to acquire an intraocular aberration, a cornea aberration, and an internal aberration of the patient's eye. On the other hand, the ophthalmologic device 10 of the third embodiment acquires measurement results of an intraocular aberration, a cornea aberration, and an internal aberration of a patient, which have been measured in past.

As illustrated in Figure 8, the ophthalmologic device 10 of the third embodiment has a configuration that is basically the same as that of the ophthalmologic device 10 of the aforementioned embodiments except that a storing unit 80 and a searching unit 82 are provided in the control device 16. Thus, like references are given to those having the same function or configuration as that of each of the aforementioned embodiments, and repetitive description is omitted. In the third embodiment, the control device 16 singly functions as an optical characteristic display device (the same is true for a fourth embodiment, which is described later).

The storing unit 80 corresponds to the first storing unit of the presently disclosed subject matter and stores past data 81 including measurement results of an intraocular aberration, a cornea aberration, and an internal aberration for each patient, which have been measured in past. This past data 81 is associated with, for example, a patient identification (ID) that is unique identification information on each of patients and an intraocular aberration, a cornea aberration, and an internal aberration for the patient. The storing unit 80 may be provided outside of (for example, in a server external to) the control device 16.

The searching unit 82 corresponds to the aberration acquiring unit of the presently disclosed subject matter and acquires an intraocular aberration, a cornea aberration, and an internal aberration corresponding to a patient ID from the past data 81 within the storing unit 80.

For example, an ID input field (not illustrated) for inputting a patient ID is provided in the optical characteristic screen 40, and when a tester inputs a patient ID to the ID input field as illustrated in Figure 9 (step S1A), the searching unit 82 acquires an intraocular aberration, a cornea aberration, and an internal aberration corresponding to the patient ID with reference to the past data 81 within the storing unit 80 (steps S1B and S1C).

In this way, the acquisition of an intraocular aberration, a cornea aberration, and an internal aberration by the searching unit 82 enables the processing in step S6 and subsequent steps illustrated in Figure 5 of the aforementioned first embodiment or the processing in step S6 and subsequent steps illustrated in Figure 7 of the aforementioned second embodiment.

According to the third embodiment as described above, an intraocular aberration, a cornea aberration, and an internal aberration are acquired from the past data 81 within the storing unit 80. Therefore, simulation for an internal aberration and an intraocular aberration of the patient's eye and simulation display of an optical characteristic of the patient's eye can be implemented without capturing the Hartmann image 23 and the corneal Meyer image 27 by the wavefront sensor 12. As a result, the time and labor of the tester and the burden on the patient can be reduced. Also, the aforementioned simulation and simulation display can be implemented in a location different from the location where the aberrations of the patient's eye are measured.

### [Fourth Embodiment]

Figure 10 is a block diagram illustrating an ophthalmologic device 10 according to a fourth embodiment. According to the third embodiment, the past data 81 representing measurement results of an intraocular aberration, a cornea aberration, and an internal aberration of a patient are stored in the storing unit 80 in the ophthalmologic device 10. On the contrary, in the fourth embodiment, the past data 81A different form the past data 81 are stored within the storing unit 80 in the ophthalmologic device 10 as illustrated in Figure 10.

Since the ophthalmologic device 10 in the fourth embodiment has a configuration that is basically the same as that of the ophthalmologic device 10 in the aforementioned third embodiment except that the past data 81A are stored within the storing unit 80, like references are given to those having the same function or configuration as that of each of the aforementioned embodiments, and repetitive description is omitted. Here, the storing unit 80 of the fourth embodiment corresponds to the second storing unit of the presently disclosed subject matter.

The past data 81A is associated with, for example, a patient ID of each patient and information (called "calculation information" below) required for calculation of each of aberrations (intraocular aberration, cornea aberration, and internal aberration) for each patient. The calculation information may include, for example, the Hartmann image 23 of a patient's eye, the corneal Meyer image 27 of a patient's eye, a centroid position and an analytic center of aberration calculations of the Hartmann image 23, or placido ring.

The searching unit 82 of the fourth embodiment constitutes the aberration acquiring unit of the presently disclosed subject matter along with the aberration calculating unit 32. When a tester inputs a patient ID to an ID input field of the optical characteristic screen 40, the searching unit 82 acquires calculation information corresponding to the patient ID with reference to the past data 81A in the storing unit 80. Then, the searching unit 82 outputs the acquired calculation information to the aberration calculating unit 32.

The aberration calculating unit 32 (intraocular aberration calculating unit 32a, cornea aberration calculating unit 32b, and internal aberration calculating unit 32c) of the fourth embodiment calculates an intraocular aberration, a cornea aberration, and an internal aberration of the patient's eye based on the calculation information input from the searching unit 82, like the aforementioned first embodiment and second embodiment. This, like the third embodiment, enables the processing in step S6 and subsequent steps illustrated in Figure 5 of the aforementioned first embodiment or the processing in step S6 and subsequent steps illustrated in Figure 7 of the aforementioned second embodiment.

According to the fourth embodiment as described above, calculation information is acquired from the storing unit 80 (past data 81A). Therefore, simulation for an internal aberration and an intraocular aberration of the patient's eye and simulation display of an optical characteristic of the patient's eye can be implemented without capturing the Hartmann image 23 and the corneal Meyer image 27 by the wavefront sensor 12. As a result, the same effect as that of the aforementioned third embodiment can be achieved.

### [Fifth Embodiment]

Figure 11 is a diagram illustrating an example of an optical characteristic screen 40 according to a fifth embodiment which allows changing operation of the diopter of an IOL as a parameter of an internal aberration. Figure 12 is a diagram illustrating an example of the optical characteristic screen 40 according to the fifth embodiment which allows changing operation of the position of an IOL as a parameter of an internal aberration.

While simulation display of an optical characteristic of a patient's eye is implemented when the axis angle of an internal aberration is changed as an internal aberration parameter according to the aforementioned embodiments, an ophthalmologic device 10 of the fifth embodiment displays a simulation result of an optical characteristic of a patient's eye when a parameter other than the axis angle, such as the diopter and the position of the IOL, for example, are changed. Since the ophthalmologic device 10 in the fifth embodiment has basically the same configuration as that of the ophthalmologic device 10 in the aforementioned embodiments, like references are given to those having the same function or configuration as that of each of the aforementioned embodiments, and repetitive description is omitted.

As illustrated in Figure 11, a display controlling unit 34 of the fifth embodiment causes a checkbox 70A to be displayed on the optical characteristic screen 40 in a mode where a simulation result of an optical characteristic of a patient's eye is displayed when the diopter of the IOL inside the patient's eye is changed (that is, the IOL is replaced).

The checkbox 70A receives a start operation (check operation) for starting a simulation display of an optical characteristic of the patient's eye when the diopter of an IOL inside the patient's eye is changed. When the checkbox 70A is checked by a check operation of a tester, the display controlling unit 34 causes an operation icon 72A and diopter information 74A to be displayed within the optical characteristic screen 40.

The operation icon 72A (corresponding to the first operating unit) receives a changing operation for changing the diopter of the IOL. This changing operation for the diopter of the IOL includes an operation for increasing the diopter of the IOL and an operation for reducing the diopter of the IOL.

The diopter information 74A displays a value of the diopter of the IOL (corresponding to a value of the parameter of the presently disclosed subject matter). Here, the display controlling unit 34 changes the display aspect of the diopter information 74A before and after the changing operation for the diopter of the IOL, like the aforementioned embodiments.

Also, as illustrated in Figure 12, the display controlling unit 34 of the fifth embodiment causes the optical characteristic screen 40 to be displayed in a mode where a simulation result of an optical characteristic of a patient's eye is displayed when the position of the IOL inside the patient's eye is changed.

The checkbox 70B receives a start operation (check operation) for starting a simulation display of an optical characteristic of the patient's eye when the position of an IOL inside the patient's eye is changed. When the checkbox 70B is checked by a check operation of a tester, the display controlling unit 34 causes an operation icon 72B and position information 74B to be displayed within the optical characteristic screen 40.

The operation icon 72B (corresponding to the first operating unit) receives a changing operation for changing (decentering) the position of the IOL. This changing operation for the position of the IOL includes an operation for individually changing each of X and Y positions of the IOL inside the patient's eye.

The position information 74B displays X and Y positions (XY coordinates) of the IOL inside the patient's eye. The display controlling unit 34 changes the display aspect of the position information 74B before and after the changing operation for the position of the IOL, like the aforementioned embodiments.

When the diopter of the IOL is changed by the changing operation on the operation icon 72A or when the position of the IOL is changed by the changing operation on the operation icon 72B, the internal aberration predicting unit 35 of the fifth embodiment predicts an internal aberration after the changing operation by a simulation.

For example, when the diopter of the IOL is changed by the changing operation, the internal aberration predicting unit 35 changes the second-order term corresponding to the diopter of the IOL in the Zernike polynomial that expresses the internal aberration to simulate the internal aberration after the change of the diopter of the IOL. Also, when the position of the IOL is changed by the changing operation, the internal aberration predicting unit 35 re-calculates an internal aberration to simulate the internal aberration after the change of the position of the IOL.

Subsequently, the re-calculating unit 36 re-calculates the intraocular aberration, and the display controlling unit 34 displays the simulation result of the optical characteristic of the patient's eye on the monitor, like the aforementioned embodiments.

Internal aberration parameters other than the axis angle, the diopter, and the position are also changed by the changing operations in similar manners. In this case, the internal aberration predicting unit 35 also simulates an internal aberration, the re-calculating unit 36 also re-calculates the intraocular aberration, and the display controlling unit 34 also displays the simulation result of the optical characteristic of the patient's eye.

Also, for example, displaying each of the operation icons 72, 72A, and 72B on the optical characteristic screen 40 at the same time enables the changing operations for parameters of an internal aberration in parallel and further enables simulation for the internal aberration, re-calculation of the intraocular aberration and simulation display of the optical characteristic of the patient's eye after the changes of the plurality of parameters.

Also in the fifth embodiment as described above, there can be provided determination information usable for determining by the doctor and the patient whether a reoperation of patient's eye is to be performed or not or, if the reoperation is to be performed, for determining whether the diopter and the position of the IOL are to be changed or not.

### [Others]

While, according to each of the aforementioned embodiments, the operation icons 72, 72A, and 72B displayed on the optical characteristic screen 40 are used for the changing operation for each kind of parameter of the internal aberration or the optimization icon 71 displayed on the optical characteristic screen 40 is used for the optimization operation on each kind of parameter, the input methods of the changing operations and the optimization operations are not particularly limited, and various kinds of publicly known operating units may be used.

While, according to each of the aforementioned embodiments, the wavefront sensor 12 (first image-capturing system 22, second image-capturing system 26) is used to acquire an intraocular aberration, a cornea aberration, and an internal aberration of the patient's eye, the method for acquiring each kind of aberration of the patient's eye is not particularly limited, and other publicly known method may be used for the acquisition.

While, according to each of the aforementioned embodiments, an optical characteristic corresponding to a cornea aberration of a patient's eye (such as cornea axial power map 50, K value information 52, cornea high-order aberration information 62, cornea spherical aberration information 64 or cornea astigmatism information 66) is displayed on the optical characteristic screen 40, the displaying of an optical characteristic corresponding to a cornea aberration may be omitted.

Having described each of the aforementioned embodiments with reference to a case where each aberration (intraocular aberration, cornea aberration, and internal aberration) of the patient's eye is expressed in a Zernike polynomial as an example, each aberration may be expressed by other functions such as a Taylor expansion polynomial, a spherical harmonic function.

Although each of the embodiments describes the example displaying the simulation result for an optical characteristic of a patient's eye into which a toric IOL is inserted, the presently disclosed subject matter is also applicable to other examples. The other examples may include an example displaying a simulation result for an optical characteristic of a patient's eye into which a publicly known intraocular lens other than the toric IOL is inserted.

### {Reference Signs List}

10 ophthalmologic device
12 wavefront sensor
14 monitor
16 control device
20 illumination system
22 first image-capturing system
23 Hartmann image
24 projecting system
26 second image-capturing system
27 corneal Meyer image
30 measurement control unit
32 aberration calculating unit
32a intraocular aberration calculating unit
32b cornea aberration calculating unit
32c internal aberration calculating unit
34 display controlling unit
35 internal aberration predicting unit
36 re-calculating unit
37 repetition controlling unit
38 evaluation value calculating unit
39 optimum value determining unit
40 optical characteristic screen
42 left/right eye switching button
44 layout switching button
46 display area
50 cornea axial power map
52 K value information
54 bulb-of-eye total aberration map
56 astigmatism map
58 spherical aberration map
60 high-order aberration map
62 cornea high-order aberration information
64 cornea spherical aberration information
66 cornea astigmatism information
68 Landolt ring simulation image
70, 70A, 70B checkbox
71 optimization icon
72, 72A, 72B operation icon
74 axis angle information
74A diopter information
74B position information
80 storing unit
81, 81A past data
82 searching unit

## Claims

1. An optical characteristic display device comprising:
an aberration acquiring unit configured to acquire an intraocular aberration, a cornea aberration, and an internal aberration of a patient's eye into which an intraocular lens is inserted;
a display controlling unit configured to display an optical characteristic of the patient's eye on a monitor based at least on the intraocular aberration and the internal aberration among the intraocular aberration, the cornea aberration, and the internal aberration acquired by the aberration acquiring unit;
a first operating unit configured to receive a changing operation, the changing operation changing only one or more parameters of the internal aberration within the patient's eye;
an internal aberration predicting unit configured to predict the internal aberration after the parameter is changed in accordance with the changing operation on the first operating unit; and
a re-calculating unit configured to re-calculate the intraocular aberration based on the internal aberration predicted by the internal aberration predicting unit and the cornea aberration acquired by the aberration acquiring unit,
wherein the display controlling unit updates the optical characteristic to be displayed on the monitor based on the internal aberration predicted by the internal aberration predicting unit and the intraocular aberration re-calculated by the re-calculating unit.

2. The optical characteristic display device according to claim 1, wherein, in addition to the optical characteristic, the display controlling unit displays an operation icon on the monitor, the operation icon representing the first operating unit.

3. The optical characteristic display device according to claim 1 or 2, wherein, every time an operation for changing the parameter is repeated in the first operating unit, prediction of the internal aberration by the internal aberration predicting unit, re-calculation of the intraocular aberration by the re-calculating unit, and update processing by the display controlling unit are repeated.

4. The optical characteristic display device according to any one of claims 1 to 3, comprising:
a second operating unit configured to receive an optimization operation for optimizing the parameter;
a repetition controlling unit configured to, when the optimization operation is received by the second operating unit, repeat the prediction of the internal aberration by the internal aberration predicting unit and the re-calculation of the intraocular aberration of the re-calculating unit by changing the parameter;
an evaluation value calculating unit configured to calculate, for the parameter, an evaluation value being an index of the optimization of the parameter based on the intraocular aberration resulting from the re-calculation for the parameter by the re-calculating unit; and
an optimum value determining unit configured to determine an optimum value for the parameter based on a calculation result for the evaluation value for the parameter by the evaluation value calculating unit.

5. The optical characteristic display device according to claim 4, wherein the evaluation value is a modulation transfer function or a Strehl definition.

6. The optical characteristic display device according to claim 4 or 5, wherein the display controlling unit selects the internal aberration and the intraocular aberration corresponding to the optimum value determined by the optimum value determining unit from among the internal aberration and the intraocular aberration for the parameter and updates the optical characteristic to be displayed on the monitor based on the internal aberration and the intraocular aberration corresponding to the optimum value.

7. The optical characteristic display device according to any one of claims 1 to 6, wherein the display controlling unit displays a value of the parameter on the monitor and changes a display aspect of the value of the parameter to be displayed on the monitor before and after the changing operation in the first operating unit.

8. The optical characteristic display device according to any one of claims 1 to 7, wherein the aberration acquiring unit includes:
a first image-capturing system configured to capture a Hartmann image of the patient's eye;
an intraocular aberration calculating unit configured to calculate the intraocular aberration based on the Hartmann image captured by the first image-capturing system;
a second image-capturing system configured to capture an anterior eye part image of the patient's eye onto which light in a predetermined pattern is projected;
a cornea aberration calculating unit configured to calculate the cornea aberration based on the anterior eye part image captured by the second image-capturing system; and
an internal aberration calculating unit configured to calculate the internal aberration based on a calculation result for the intraocular aberration by the intraocular aberration calculating unit and a calculation result for the cornea aberration by the cornea aberration calculating unit.

9. The optical characteristic display device according to any one of claims 1 to 7, comprising:
a first storing unit configured to store the intraocular aberration, the cornea aberration, and the internal aberration that are measured in advance,
wherein the aberration acquiring unit acquires the intraocular aberration, the cornea aberration, and the internal aberration from the first storing unit.

10. The optical characteristic display device according to any one of claims 1 to 7, comprising:
a second storing unit configured to store information that is required for calculating the intraocular aberration, the cornea aberration, and the internal aberration of the patient's eye, including the Hartmann image of the patient's eye and the anterior eye part image of the patient's eye onto which light in the predetermined pattern is projected,
wherein the aberration acquiring unit includes:
an intraocular aberration calculating unit configured to calculate the intraocular aberration based on the information stored in the second storing unit;
a cornea aberration calculating unit configured to calculate the cornea aberration based on the information stored in the second storing unit; and
an internal aberration calculating unit configured to calculate the internal aberration based on the information stored in the second storing unit.

11. The optical characteristic display device according to any one of claims 1 to 10, wherein
the parameter includes at least any one of an axis angle, a diopter, and a position of the intraocular lens.

12. An optical characteristic display method comprising:
an aberration acquiring step of acquiring an intraocular aberration, a cornea aberration, and an internal aberration that are an intraocular aberration, a cornea aberration, and an internal aberration of a patient's eye into which an intraocular lens is inserted and that are expressed by a function;
a display controlling step of displaying an optical characteristic of the patient's eye on a monitor based at least on the intraocular aberration and the internal aberration among the intraocular aberration, the cornea aberration, and the internal aberration acquired by the aberration acquiring step;
an operating step of receiving a changing operation, the changing operation only changing a parameter of the internal aberration within the patient's eye;
a predicting step of predicting the internal aberration after the parameter is changed in accordance with the changing operation in the operating step;
a re-calculating step of re-calculating the intraocular aberration based on the internal aberration predicted by the predicting step and the cornea aberration acquired by the aberration acquiring step; and
an updating step of updating the optical characteristic to be displayed on the monitor based on the internal aberration predicted by the predicting step and the intraocular aberration resulting from the re-calculation processing by the re-calculating step.
